(19) **Europäisches Patentamt / European Patent Office / Office européen des brevets**

(11) **EP 3 383 467 B1**

(12) **EUROPÄISCHE PATENTSCHRIFT**

(45) Veröffentlichungstag und Bekanntmachung des Hinweises auf die Patenterteilung:
**24.07.2019 Patentblatt 2019/30**

(21) Anmeldenummer: **16867382.0**

(22) Anmeldetag: **21.11.2016**

(51) Int Cl.:
*A61M 16/01* (2006.01)     *A61M 16/10* (2006.01)
*A61M 16/08* (2006.01)     *A61B 5/08* (2006.01)
*A61M 16/00* (2006.01)

(86) Internationale Anmeldenummer:
**PCT/EP2016/001952**

(87) Internationale Veröffentlichungsnummer:
**WO 2017/137055 (17.08.2017 Gazette 2017/33)**

(54) **ANÄSTHESIEBEATMUNGSVORRICHTUNG ZUR AUTOMATISIERTEN BEATMUNG EINES PATIENTEN**

ANAESTHESIA VENTILATION APPARATUS FOR AUTOMATICALLY VENTILATING A PATIENT

DISPOSITIF D'ANESTHÉSIE VENTILATOIRE POUR LA VENTILATION AUTOMATISÉE D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.12.2015 DE 102015015440**

(43) Veröffentlichungstag der Anmeldung:
**10.10.2018 Patentblatt 2018/41**

(73) Patentinhaber: **Drägerwerk AG & Co. KGaA 23558 Lübeck (DE)**

(72) Erfinder:
• **BUSCHKE, Wilfried 23560 Lübeck (DE)**
• **HÖRMANN, Christoph 3240 Mank (AT)**
• **MERSMANN, Stefan 23564 Lübeck (DE)**

(56) Entgegenhaltungen:
**EP-B1- 2 572 748**     **DE-A1- 19 751 597**
**DE-A1-102011 106 406**     **DE-T2- 69 630 181**
**US-A- 6 131 571**

EP 3 383 467 B1

**Beschreibung**

Aus dem Stand der Technik sind Anästhesiebeatmungsvorrichtungen sowie Verfahren bekannt, bei welchen eine Beatmung in automatisierter Weise in Abhängigkeit eines Solldruckwertes sowie eines durch einen Drucksensor erfassten Drucks eines Atemgases durchgeführt wird.

Ferner sind Verfahren bekannt, bei welchen im Rahmen einer Beatmung ein sogenanntes Weaning durchgeführt wird, bei welchem der Patient in einer sogenannten Komfortzone gehalten wird, wobei für den Zweck des Weaning im Rahmen einer druckunterstützenden Beatmung ein Solldruckwert bzw. ein Druckunterstützungswert in Abhängigkeit eines detektierten Tidalvolumens sowie einer endexspiratorischen Kohlendioxidkonzentrationswertes angepasst wird. Derartige Verfahren sind auch als sogenannte "Smart-Care/PS" Verfahren bekannt.

Die DE 696 30 181 T2 offenbart ein Anästhesiegerät, bei welchem ein Gasmesser eine Konzentration eines Gases misst und bei welchem durch eine Gasregeleinheit die einzelnen Gasbestandteile in spezifischen Verhältnissen gemischt werden, wobei die Gasregeleinheit auch den Druck und den Fluss des Atemgases regelt.

Aus der US 6,131,571 ist ein Anästhesiegasgerät bekannt, bei welchem ein Multigassensor ein Vorhandensein von Sauerstoff, eines Anästhesiegas, Kohlendioxid sowie anderer Gase misst, sodass eine Kontrolleinheit erste, zweite sowie dritte Zuführungseinheiten in Abhängigkeit der als vorhanden gemessenen Gase kontrolliert. Diese unterschiedlichen Zuführungseinheiten führen Sauerstoff, ein zweites Gas sowie das Anästhesiegas in der Weise zu, dass gewünschte, relative Konzentrationen von Sauerstoff, dem zweiten Gas sowie des Anästhesiegases erreicht werden.

Aus der EP 2 572 748 B1 ist ein Anästhesiegerät bekannt, bei welchem eine Kontrolleinheit einen Kompressor in Abhängigkeit eines gemessenen Drucks derart ansteuert, dass ein tatsächlicher Druck einem Zieldruckwert entspricht. Ferner steuert die Kontrolleinheit ein Regulierungsventil an, welches einen Fluss eines Anästhesiemittels in Abhängigkeit eines Signals eines Anästhesiegassensors reguliert.

Aus der DE 197 51 597 A1 ist ein Anästhesiebeatmungsgerät bekannt, bei welchem eine mobile und leicht nachfüllbare Sauerstoffquelle in Form von mehreren Chloratkerzen mit zugehörigen Zündeinrichtungen vorgesehen ist, wobei mittels einer Steuereinheit eine vorbestimmte Sauerstoffkonzentration eingestellt wird.

Die DE 10 2011 106 406 A1 offenbart ein Verfahren zur Steuerung eines Anästhesie- und Beatmungsgerätes in der Ausatemphase eines Patienten, wobei der Druck in der Ausatemphase auf einen positiv-endexspiratorischen Druck ausgeregelt wird und wobei der positiv-endexspiratorische Druck einen über der Exspirationsphase fallenden Verlauf hat. Der fallende Verlauf des positiv-endexspiratorischen Drucks in der Ausatemphase des Patienten wird vorzugsweise aus den Einstellwerten für die Beatmung des Patienten ermittelt. Aufgabe der vorliegenden Erfindung ist es, eine Anästhesiebeatmung eines Patienten in automatisierter Weise durchzuführen, wobei ein vorgegebener Solldruckwert für die Beatmung berücksichtigt wird und wobei die Beatmung derart durchgeführt wird, dass die Beatmung des Patienten in vorteilhafter Weise angepasst wird.

Die erfindungsgemäße Aufgabe wird gelöst durch eine Anästhesiebeatmungsvorrichtung nach dem Patentanspruch 1. Ferner wird die erfindungsgemäße Aufgabe gelöst durch eine Recheneinheit nach dem Patentanspruch 9.

Vorteilhafte Ausführungsformen der Erfindung sind Gegenstand der abhängigen Ansprüche und werden in der folgenden Beschreibung unter teilweiser Bezugnahme auf die Figuren näher erläutert.

Vorgeschlagen wird eine Anästhesiebeatmungsvorrichtung zur automatisierten Beatmung eines Patienten, aufweisend einen exspiratorischen Port und einen inspiratorischen Port zum Anschluss eines dem Patienten zugewandten Beatmungsschlauches für ein Atemgas, eine Atemgasfördereinheit, wenigstens einen Atemgassensor zum Erfassen einer Anästhesiegaskonzentration, wenigstens einen Drucksensor zum Erfassen eines Drucks des Atemgases, sowie wenigstens eine Recheneinheit, wobei die Recheneinheit dazu ausgebildet ist, die Atemgasfördereinheit in Abhängigkeit des erfassten Drucks und eines vorgegebenen Solldruckwertes anzusteuern und wobei die Recheneinheit ferner dazu ausgebildet ist, eine Anpassung des Solldruckwertes in Abhängigkeit der erfassten Anästhesiegaskonzentration vorzunehmen. Die vorgeschlagene Anästhesiebeatmungsvorrichtung ist vorteilhaft, da aufgrund der Anpassung des Solldruckwertes in Abhängigkeit der erfassten Anästhesiegaskonzentration die Beatmung des Patienten an die aktuelle Anästhesiesituation angepasst werden kann.

Vorzugsweise ist die Recheneinheit dazu ausgebildet, auf Basis der erfassten Anästhesiegaskonzentration eine mittlere alveoläre Anästhesiegaskonzentration sowie eine endexspiratorische Anästhesiegaskonzentration zu ermitteln, und ferner eine Anpassung des Solldruckwertes in Abhängigkeit der ermittelten mittleren alveolären Anästhesiegaskonzentration sowie in Abhängigkeit der ermittelten endexspiratorischen Anästhesiegaskonzentration vorzunehmen. Diese Ausgestaltung der Erfindung ist vorteilhaft, da nicht nur eine mittlere alveolären Anästhesiegaskonzentration als Hinweis auf ein prinzipielles Vorhandensein von Anästhesiegas im Atemgas berücksichtigt wird, sondern auch eine Anästhesiegaskonzentration in der endexspiratorischen Phase, welche Aufschluss darüber gibt, zu welchem Grad der Patient das Anästhetikum mittels seines Stoffwechsels abbaut. Somit kann durch Berücksichtigung dieser zwei Größen hinsichtlich des Anästhetikums der Solldruckwert in genauerer Art und Weise angepasst werden kann. Beispielsweise ist es möglich, dass zwar die mittlere alveolären Anästhesiegaskonzentration zeitlich abnimmt, dass aber aufgrund eines unzureichenden Stoffwechsels das Anästhetikums durch den Patienten die endexspiratorische Anästhesiegaskonzentration nicht wie gewünscht weiter abnimmt, sodass durch Anpassung des Solldruckwertes in Abhängigkeit der beiden Konzentrationen die Beatmung des Patienten hierauf angepasst werden kann.

Vorzugsweise weist die Vorrichtung ferner wenigstens einen Volumenstromsensor zum Erfassen eines Volumenstroms des Atemgases sowie ferner wenigstens einen Atemgassensor zum Erfassen einer Kohlendioxidkonzentration des Atemgases auf, wobei die Recheneinheit ferner dazu ausgebildet ist, eine Anpassung des Solldruckwertes sowie einer minimalen Beatmungsfrequenz in Abhängigkeit des erfassten Volumenstromes und in Abhängigkeit der erfassten Kohlendioxidkonzentration vorzunehmen. Diese Ausgestaltung der Erfindung ist vorteilhaft, da der Solldruckwert und die minimale Beatmungsfrequenz in noch genauerer Weise angepasst werden kann, da nicht nur eine Anästhesiegaskonzentration hierzu in Betracht gezogen wird, sondern auch die Kohlendioxidkonzentration des Atemgases als auch der Volumenstrom, welcher ein Tidalvolumen des Patienten indiziert.

Vorzugsweise ist die Recheneinheit dazu ausgebildet, auf Basis des erfassten Volumenstroms ein dem Patienten zugeführtes Tidalvolumen zu bestimmen und ferner auf Basis der erfassten Kohlendioxidkonzentration eine endexspiratorische Kohlendioxidkonzentration zu bestimmen, wobei die Recheneinheit ferner dazu ausgebildet ist, die Anpassung des Solldruckwertes ferner in Abhängigkeit des bestimmten Tidalvolumens, eines oberen Volumengrenzwertes, eines unteren Volumengrenzwertes, der bestimmten endendexspiratorischen Kohlendioxidkonzentration, eines oberen Konzentrationsgrenzwertes und eines unteren Konzentrationsgrenzwertes vorzunehmen. Diese Ausgestaltung der Erfindung ist vorteilhaft, da durch die jeweiligen Grenzwerte bezogen auf das Tidalvolumen bzw. bezogen auf die endendexspiratorische Kohlendioxidkonzentration eine sogenannte Komfortzone definiert wird, wobei durch Vergleich der Grenzwerte dieser Komfortzone und dem Tidalvolumen einerseits als auch der endendexspiratorischen Kohlendioxidkonzentration andererseits die Anpassung der Beatmungsfrequenz und des Solldruckwertes vorgenommen wird, um den Patienten so möglicherweise während der Beatmung innerhalb dieser Komfortzone zu behalten bzw. zu führen.

Vorzugsweise ist die Recheneinheit dazu ausgebildet, in Abhängigkeit des bestimmten Tidalvolumens, der bestimmten endendexspiratorischen Kohlendioxidkonzentration, eines weiteren vorgegebenen Volumengrenzwertes, eines weiteren vorgegebenen Konzentrationsgrenzwertes, des aktuellen Solldruckwertes sowie eines vorgegebenen Solldruckgrenzwertes einen gewünschten Betriebszustand hinsichtlich der automatisierten Beatmung zu detektieren und bei Detektion ein Ausgabesignal an einen Kliniker auszugeben, welches ein Vorliegen des gewünschten Betriebszustandes indiziert. Ein solcher Betriebszustand ist dann gegeben, wenn das Tidalvolumen und die endexspiratorische Kohlendioxidkonzentration jeweilige gewünschte Korrelationen zu den jeweiligen Grenzwerten aufweisen. Liegt der Betriebszustand vor, so kann dies ein Hinweis für einen Kliniker sein, dass der Patient möglicherweise ein gewünschtes Atemverhalten aufweist. Die Ausgabe des Ausgabesignals, welches das Vorliegen des Betriebszustandes der Vorrichtung hinsichtlich der automatisierten Beatmung indiziert, kann dann für einen Kliniker ein Hinweis sein, ein Extubieren des Patienten in Betracht zu ziehen. Eine solche Situation liegt beispielsweise dann vor, wenn im Rahmen einer Anästhesiebeatmung die Zuführung von Anästhesiegasen durch eine Narkosegasmischeinheit innerhalb des Beatmungsgerätes durch den Kliniker zuvor unterbunden wurde und der Kliniker dann in der Endphase der Anästhesiebeatmung evtl. ein Extubieren in Betracht ziehen möchte.

Vorzugsweise ist die Recheneinheit dazu ausgebildet, die Atemgasfördereinheit so anzusteuern, dass die automatisierte Beatmung als eine druckunterstützende Beatmung durchgeführt wird. Diese Ausgestaltung der Erfindung ist vorteilhaft, da eine druckunterstützende Beatmung eine Spontanatemaktivität eines Patienten erfordert, welches insbesondere in Endphasen einer Anästhesiebeatmung gewünscht ist.

Vorzugsweise weist die Vorrichtung ferner wenigstens einen Volumenstromsensor zum Erfassen eines Volumenstroms des Atemgases auf, wobei die Recheneinheit ist ferner dazu ausgebildet, auf Basis des erfassten Volumenstroms einen Spontanatemversuch des Patienten zu detektieren, und ferner die druckunterstützende Beatmung unter Verwendung des Solldruckwertes bei Detektion eines Spontanatemversuchs durchzuführen. Diese Ausgestaltung der Erfindung ist vorteilhaft, da hierdurch die Spontanatemaktivität eines Patienten detektiert werden kann.

Vorzugsweise ist die Recheneinheit ferner dazu ausgebildet, in Abhängigkeit detektierter Spontanatemversuche und in Abhängigkeit einer vorgebbaren minimalen Beatmungsfrequenz eine Ausgabe eines Warnsignals zu steuern. Diese Ausgestaltung der Erfindung ist vorteilhaft, da bei einer möglichweise zu geringen Atemfrequenz aufgrund von eigener Spontanatemaktivität des Patienten, also unterschreiten einer Mindestatemfrequenz, der Kliniker darüber informiert wird, dass er möglicherweise selber Maßnahmen einleiten könnte, um die aktuelle Beatmungssituation des Patienten zu ändern.

Vorgeschlagen wird ferner ein Verfahren zum Betreiben einer Anästhesiebeatmungsvorrichtung für eine automatisierte Beatmung eines Patienten, aufweisend: Zuführen eines Atemgases zu einem Patienten über einen inspiratorischen Port und Rückführen des Atemgases über einen endexspiratorischen Port durch Betreiben einer Atemgasfördereinheit, Erfassen einer Anästhesiegaskonzentration mittels wenigstens eines Atemgassensors, Erfassen eines Drucks des Atemgases mittels wenigstens eines Drucksensors, Ansteuern der Atemgasfördereinheit in Abhängigkeit des erfassten Drucks und eines vorgegebenen Solldruckwertes mittels wenigstens einer Recheneinheit, gekennzeichnet durch eine Anpassung des Solldruckwertes in Abhängigkeit der erfassten Anästhesiegaskonzentration mittels der Recheneinheit.

Ferner wird eine Recheneinheit für eine Anästhesiebeatmungsvorrichtung zur automatisierten Beatmung eines Patienten vorgeschlagen, die ausgebildet ist zum Erfassen eines Anästhesiegaskonzentrationssignals, welches eine Anästhesiegaskonzentration eines Atemgases indiziert, Erfassen eines Drucksignals, welches einen Druck des Atemgases indiziert, Bereitstellen eines Ansteuersignals für eine Atemgasfördereinheit, wobei die Recheneinheit das Ansteuersignal in Abhängigkeit des erfassten Drucksignals und eines vorgegebenen Solldruckwertes bestimmt und wobei die Recheneinheit ferner dazu ausgebildet ist, eine Anpassung des Solldruckwertes in Abhängigkeit des erfassten Anästhesiegaskonzentrationssignals vorzunehmen.

Ferner wird ein Verfahren zum Betreiben einer Anästhesiebeatmungsvorrichtung zur automatisierten Beatmung eines Patienten vorgeschlagen, aufweisend: Erfassen eines Anästhesiegaskonzentrationssignals, welches eine Anästhesiegaskonzentration eines Atemgases indiziert, Erfassen eines Drucksignals, welches einen Druck des Atemgases indiziert, Bereitstellen eines Ansteuersignals für eine Atemgasfördereinheit in Abhängigkeit des erfassten Drucksignals und eines vorgegebenen Solldruckwertes, gekennzeichnet durch eine Anpassung des Solldruckwertes in Abhängigkeit des erfassten Anästhesiegaskonzentrationssignals.

Ferner wird das zuvor genannte Verfahren zum Betreiben einer Anästhesiebeatmungsvorrichtung zur automatisierten Beatmung eines Patienten vorgeschlagen, wobei das Verfahren mit Computerprogrammmitteln auf wenigstens einer Recheneinheit ausgeführt wird.

Vorteile der vorgeschlagenen Anästhesiebeatmungsvorrichtung gelten ebenso für das vorgeschlagene Verfahren zur automatisierten Beatmung eines Patienten. Ebenso gelten diese Vorteile für die vorgeschlagene Recheneinheit für eine Anästhesiebeatmungsvorrichtung. Ebenso gelten diese Vorteile auch für ein Verfahren zum Betreiben einer Anästhesiebeatmungsvorrichtung. Im Folgenden wird die Erfindung anhand spezieller Ausführungsformen ohne Beschränkung des allgemeinen Erfindungsgedankens anhand der Figuren näher erläutert. Dabei zeigen:

Fig. 1     einen zeitlichen Druckverlauf sowie einen zeitlichen Volumenstromverlauf im Zuge einer Inspiration und einer Exspiration,

Fig. 2     einen zeitlichen Druckverlauf sowie einen zeitlichen Volumenstromverlauf im Rahmen einer druckunterstützenden Beatmung, bei welcher Spontanatemversuche eines Patienten ermöglicht werden,

Fig. 3     eine erfindungsgemäße Anästhesiebeatmungsvorrichtung,

Fig. 4     Schritte des vorgeschlagenen Verfahrens,

Fig. 5     erste Teilschritte des vorgeschlagenen Verfahrens,

Fig. 6     zweite Teilschritte des vorgeschlagenen Verfahrens,

Fig. 7     eine Illustration von Grenzwerten,

Fig. 8     eine Tabelle mit durch einen Kliniker vorgebbaren Vorgabewerten,

Fig. 9     eine Tabelle mit möglichen Grenzwerten zur Durchführung des vorgeschlagenen Verfahrens,

Fig. 10    jeweilige Tabellen zur Bestimmung von Ventilationsgraden bzw. Gasaustauschraten bezogen auf ein Tidalvolumen bzw. eine endendexspiratorische Kohlendioxidkonzentration,

Fig. 11    eine Tabelle mit Werten zur Anpassung des Solldruckwertes und zur Anpassung der minimalen Beatmungsfrequenz,

Fig. 12a   einen zeitlichen Verlauf einer mittleren alveolären Anästhesiegaskonzentration,

Fig. 12b   einen zeitlichen Verlauf einer endexspiratorischen Anästhesiegaskonzentration und

Fig. 13    Grenzwerte zur Detektion eines Betriebszustandes hinsichtlich der automatisierten Beatmung,

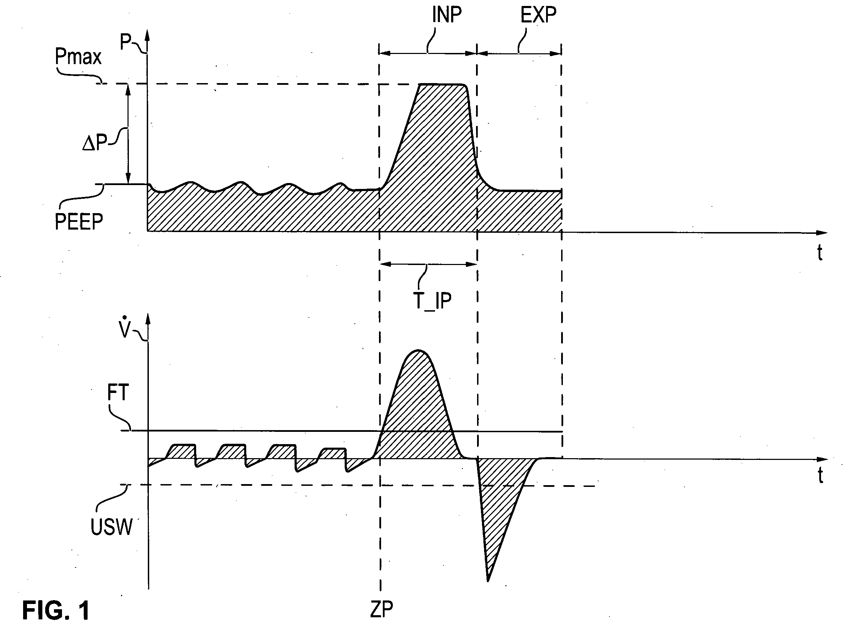

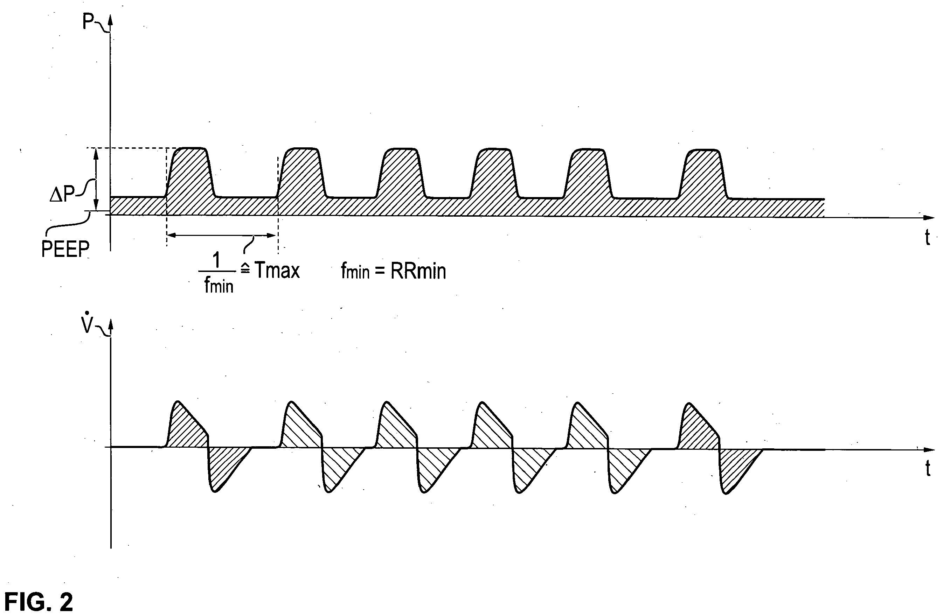

Figuren 1 und 2 zeigen aus dem Stand der Technik bekannte Kurvenverläufe, anhand derer nun die Prinzipien einer Triggersteuerung im Zuge einer druckunterstützenden Beatmung näher erläutert werden. Diese Prinzipien sind auch dem Dokument

- Beatmungsmodi in der Intensivmedizin", Karin Deden, Dräger Medical GmbH, sowie
- Gebrauchsanweisung "Zeus Infinity Empowered", Dräger Medical AG & Co. KG, 1. Ausgabe, Februar 2009 entnehmbar.

Die Figur 1 zeigt zur Illustration einer Triggersteuerung einen Druckverlauf eines Druckwertes P über der Zeit, wobei ferner ein zeitlicher Verlauf eines Volumenstroms V zu sehen ist. Wird ein Patient durch eine Anästhesiebeatmungsvorrichtung beatmet, so erfolgt eine Druckunterstützung derart, dass vor einer inspiratorischen Phase INSP der Druck auf einen mindesten endendexspiratorischen Druck PEEP (Positive End Exspiratory Pressure) gehalten wird. Macht ein Patient einen Spontanatemversuch, so führt dies zum Überschreiten des Volumenstroms V über eine sogenannte Triggerschwelle bzw. Flow-Triggerschwelle FT zum Zeitpunkt ZP. Es wird dann bei Schwellenüberschreitung der Druck P derart gesteuert, dass der Druck P auf einen Maximaldruck Pmax gesteuert wird, wobei dieser Maximaldruck Pmax um einen Differenzdruck $\Delta P$ über dem Mindestdruck PEEP liegt. Üblicherweise wird eine Zeitdauer T_IP für eine inspiratorische Phase vorgegeben, sodass dann nach Ablauf der Zeitdauer T_IP mit der endexspiratorischen Phase EXP begonnen wird, während welcher wiederum der Druck P auf den Minimaldruck PEEP abgesenkt wird. Somit ergibt sich während der endexspiratorischen Phase aufgrund des Ausströmens des Volumenstromes V aus dem Patienten heraus ein negativer Volumenstrom V.

Eine derartige triggergesteuerte Beatmung wird üblicherweise im Rahmen von einer druckunterstützenden Beatmung durchgeführt, wie sie auch noch einmal in der Figur 2 gezeigt ist. Eine solche druckunterstützende Beatmung wird ferner vorzugsweise derart durchgeführt, dass der Patient aufgrund seiner durch ihn initiierten Triggerung eine Mindestatemfrequenz fmin bzw. RRmin herbeiführen muss, sodass sich ein maximales Zeitfenster Tmax zwischen zwei Anfangszeitpunkten benachbarter inspiratorischer Phasen ergibt. Führt die durch den Patienten getriggerte Beatmung dazu, dass nach Ablauf des Zeitfensters Tmax keine erneute Inspiration durchgeführt wird, so kann in einem solchen Fall eine Warnung ausgegeben werden. Ist also die Atemfrequenz der Spontanatmung des Patienten geringer als die minimale Beatmungsfrequenz fmin bzw. RRmin, so wird eine Warnung ausgegeben, welche eine zu geringe Spontanatmung des Patienten indiziert.

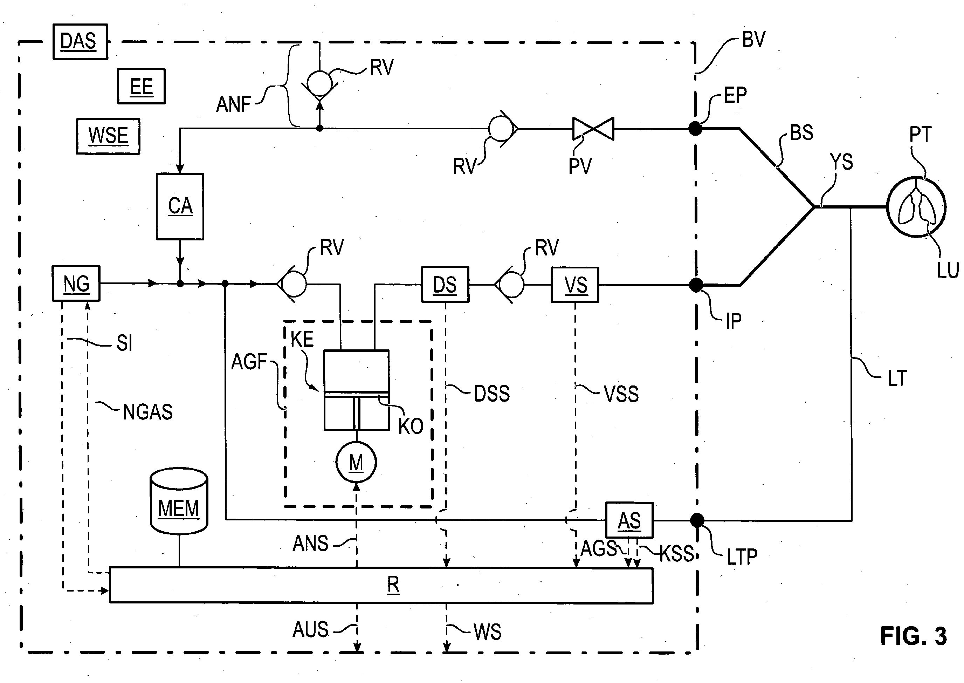

Die Figur 3 zeigt die erfindungsgemäße Vorrichtung BV zur automatisierten Beatmung eines Patienten P. Die erfindungsgemäße Anästhesiebeatmungsvorrichtung BV weist einen inspiratorischen Port IP und einen endexspiratorischen Port EP auf, an welchen ein Beatmungsschlauch BS angeschlossen werden kann, welcher dem Patienten PT zugewandt ist. Über diesen Beatmungsschlauch BS wird ein Atemgas dem Patienten zugeführt und auch wieder von dem Patienten hin zur Vorrichtung BV abgeführt. Die Zuführung erfolgt über den inspiratorischen Port IP, die Abführung erfolgt über den endexspiratorischen Port EP. Der Beatmungsschlauch BS führt die Anschlüsse der Ports EP, IP an einem sogenannten Y-Stück YS zusammen, welches dann üblicherweise an einem Tubus endet, der dem Patienten PT eingeführt wird, um ihn über seine Lunge LU zu beatmen.

Die Anästhesiebeatmungsvorrichtung BV weist ferner eine Atemgasfördereinheit AGF auf. Vorzugsweise ist die Atemgasfördereinheit AGF eine Kolbeneinheit KE, in welcher ein Kolben KO durch einen Motor M vor- bzw. zurückgeführt werden kann.

Die Vorrichtung BV weist wenigstens eine Recheneinheit R auf.

Bei der Recheneinheit R handelt es sich um wenigstens eine Recheneinheit, welche auch durch einen Verbund mehrerer Recheneinheiten verwirklicht werden kann.

Ferner weist die Anästhesiebeatmungsvorrichtung BV einen Drucksensor DS zum Erfassen eines Drucks des Atemgases auf. Der Drucksensor DS stellt ein Drucksensorsignal DSS an die Recheneinheit R bereit.

Ein Mindestdruck PEEP wird vorzugsweise durch ein Ventil PV, welches vorzugsweise in dem Bereich des endexspiratorischen Ports EP liegt, realisiert.

Die Anästhesiebeatmungsvorrichtung BV weist ferner einen Atemgassensor AS auf. Der Atemgassensor AS ist ausgebildet zum Erfassen einer Anästhesiegaskonzentration des Atemgases auf. Der Atemgassensor AS stellt ein Anästhesiegaskonzentrationssignal AGS an die Recheneinheit R bereit. Ferner ist der Atemgassensor AS vorzugsweise ausgebildet zum Erfassen einer Kohlendioxidkonzentration des Atemgases. Der Atemgassensor AS stellt vorzugsweise ein Kohlendioxidkonzentrationssignal KSS an die Recheneinheit R bereit. Der Atemgassensor AS ist vorzugsweise kein einzelner Sensor sondern eine Sensoreinheit, welche mehrere, jeweils speziell ausgebildete Sensoren zur Erfassung der jeweiligen zuvor genannten Konzentrationen aufweist.

Der Sensor AS ist vorzugsweise hinter einer Messleitung LT vorgesehen, welche eine Messprobe des Atemgases am Y-Stück YS abführt und an einen Messgasport LTP angeschlossen ist.

Die Anästhesiebeatmungsvorrichtung BV weist einen Kohlendioxidabsorber CA sowie eine Narkosegasmischeinheit NG auf. Über die Narkosegasmischeinheit NG kann dann ein für die Narkose erforderliches Gasgemisch in den Atemkreislauf eingebracht werden. Ein solches Gasgemisch weist dann wenigstens ein Anästhetikum auf.

Ferner weist die Anästhesiebeatmungs-vorrichtung eine Anästhesiegasfortleitung ANF bzw. einen Anschluss an eine Anästhesiegasfortleitung ANF auf. Der Gasfluss innerhalb der Anästhesiebeatmungsvorrichtung BV ist vorzugsweise durch Rückschlagventile RV kontrolliert. Die Recheneinheit R steuert die Narkosegasmischeinheit NG mittels eines Steuersignals NGAS. Die Narkosemischeinheit NG stellt vorzugsweise ein Statussignal SI an die Recheneinheit R bereit, welches indiziert, ob die Narkosemischeinheit NG ein Anästhetikum in das Atemgas einbringt oder nicht. Vorzugsweise indiziert dieses Statussignal, ob ein Vapor geöffnet ist oder nicht.

Die Anästhesiebeatmungsvorrichtung BV weist vorzugsweise wenigstens einen Volumenstromsensor VS zum Erfassen eines Volumenstroms des Atemgases auf. Der Volumenstromsensor VS stellt ein Volumenstromsensorsignal VSS an die Recheneinheit R bereit.

Die Anästhesiebeatmungsvorrichtung BV aus Figur 3 weist vorzugsweise eine Eingabeeinheit EE oder eine Schnittstelle EE zu einer Eingabeeinheit auf, mittels welcher Eingaben an der Anästhesiebeatmungsvorrichtung BV entgegengenommen werden können, welche durch einen Bediener bzw. Kliniker an der Eingabeinheit vorgenommen werden können.

Die Recheneinheit R greift vorzugsweise auf eine Speichereinheit MEM zurück, um das vorgeschlagenen Verfahren durchzuführen.

Die Recheneinheit R gibt vorzugsweise ein Warnsignal WS in der zuvor beschriebenen Weise aus, um ein Vorliegen einer Beatmungsfrequenz, welche die minimale Beatmungsfrequenz unterschreitet, zu indizieren. Diese Ausgabe erfolgt vorzugsweise über eine Datenschnittstelle DAS der Vorrichtung BV. Vorzugsweise weist die Vorrichtung BV selber eine Warnsignalausgabeeinheit WSE auf, welche vorzugsweise eine optische und/oder akustische Warnung ausgeben kann.

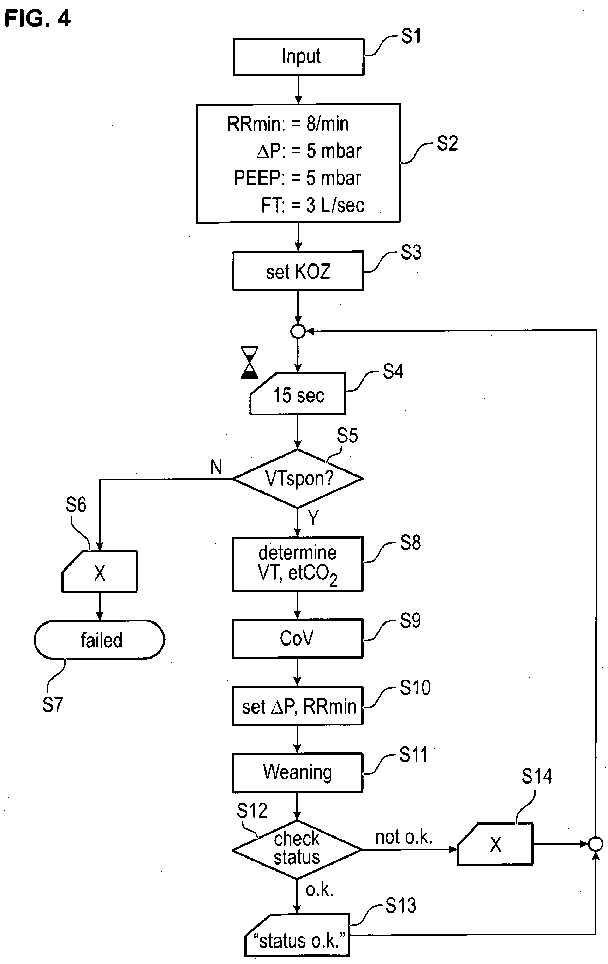

Die erfindungsgemäße Anästhesiebeatmungsvorrichtung ist vorzugsweise dazu ausgebildet, eine druckunterstützende Beatmung des Patienten PT durchzuführen. Figur 4 zeigt Schritte, mittels derer die Anästhesiebeatmungsvorrichtung aus Figur 3 zur Durchführung des vorgeschlagenen Verfahrens veranlasst werden kann.

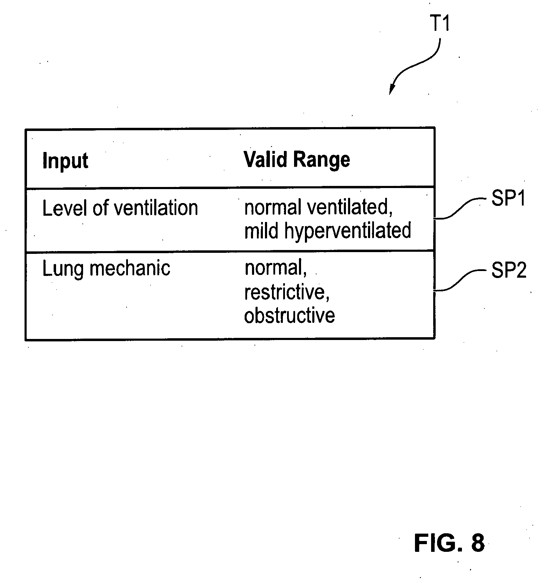

Figur 8 zeigt in der Tabelle T1 Möglichkeiten verschiedener Eingaben, welche im Rahmen des Schrittes S1 der Figur 4 durch einen Kliniker eingegeben werden können. In der ersten Spalte SP1 finden sich verschiedene Einträge bzw. Vorgaben, mittels derer ein gewünschter Ventilationsgrad bzw. eine gewünschte Gasaustauschrate eines Patienten ausgewählt werden kann. Hierbei handelt es sich vorzugsweise um die Varianten einer normalen Ventilation bzw. einer milden Hyperventilation. Derartige Eingaben können durch die in Figur 3 gezeigte Schnittstelle EE bzw. Eingabeeinheit EE der Anästhesiebeatmungsvorrichtung BV entgegengenommen werden. Diese Eingabeeinheit EE kann vorzugsweise eine zu der Anästhesiebeatmungsvorrichtung BV zugehörige oder eine mit der Anästhesiebeatmungsvorrichtung BV in Kommunikation stehende Eingabeeinheit in Form einer Tastatur, eines Touch-Screens und/oder einer Computermaus sein.

In Figur 8 finden sich in der zweiten Spalte SP2 ferner verschiedene Möglichkeiten von Vorgaben bezüglich einer Lungeneigenschaft ("Lung Mechanic") des Patienten. Die Vorgabe kann eine Lungeneigenschaft kann eine normale, eine restriktive oder eine obstruktive Patientenlunge indizieren. Zurückkommend zur Figur 4 kann nun in dem Schritt S2 eine Initialisierung von beatmungsrelevanten Parametern vorgenommen werden. Hierbei wird die minimale Beatmungsfrequenz RRmin vorzugsweise auf 8 Atemzüge/min gesetzt, die Druckdifferenz $\Delta P$ auf 5 mbar, der Mindestdruck PEEP auf 5 mbar und die Triggerschwelle FT auf 3 Liter/s. Es ist für einen Fachmann klar und erkennbar, dass die hier gezeigten Werte nur exemplarische Werte sind und bei Ausführung des vorgeschlagenen Verfahrens sowie Realisierung der erfindungsgemäßen Vorrichtung auch anders gewählt werden können.

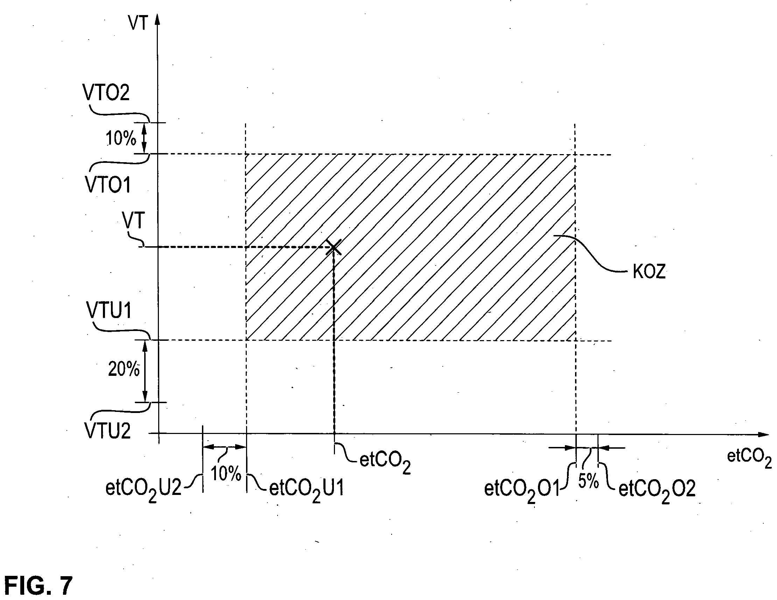

In einem Schritt S3 werden vorzugsweise verschiedene Grenzwerte initialisiert, welche als eine sogenannte Komfortzone KOZ aufgefasst werden können. Hierzu lässt sich in Korrespondenz zu diesem Schritt S3 die Figur 7 betrachten. Die Figur 7 indiziert die Komfortzone KOZ, welche immer dann gegeben ist, wenn aus einem erfassten Volumenstrom durch die Recheneinheit ein durch den Patienten eingeatmetes Tidalvolumen VT ermittelt wird, welches zwischen einem oberen Volumengrenzwert VTO1 und einem unteren Volumengrenzwert VTU1 liegt. Ferner ist es zum Erreichen der Komfortzone KOZ erforderlich, dass eine durch Recheneinheit R auf Basis des Kohlendioxidkonzentrationssignals KSS ermittelte endexspiratorische Kohlendioxidkonzentration etCO2 vorliegt, welche zwischen einem oberen Konzentrationsgrenzwert etCO2O1 und einem unteren Konzentrationsgrenzwert etCO2U1 liegt.

Ein vorzugsweise zu erreichendes Ziel des Verfahrens ist es, den Patienten derart zu beatmen, dass durch die Beatmung der Patient ein Tidalvolumen VT aufweist bzw. atmet, welches innerhalb der Volumengrenzwerte VTO1, VTU1 liegt und dass der Patient gleichzeitig auch eine endexspiratorische Kohlendioxidkonzentration etCO2 aufweist, welche innerhalb der Konzentrationsgrenzwerte etCO2U1 und etCO2O1 liegt.

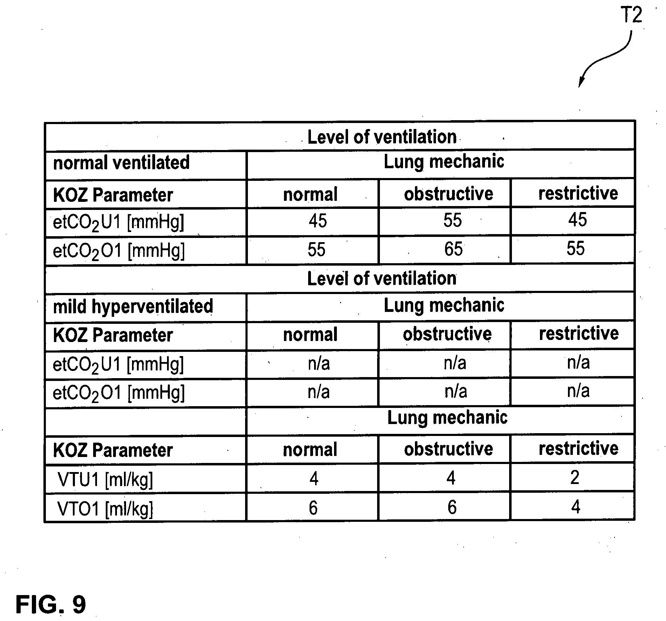

In Bezug auf den Schritt S3 aus Figur 4 kann unter Hinzunahme der Figur 9 und unter Betrachtung der Tabelle T2 entnommen werden, in welcher Weise durch Auswahl der Vorgaben hinsichtlich der Lungeneigenschaft des Patienten sowie der Gasaustauschrate bzw. des Ventilationsgrades die jeweiligen Volumengrenzwerte VTO1, VTU1 sowie die jeweiligen Konzentrationsgrenzwerte etCO2U1, etCO2O1 gewählt werden können. Hierbei ist in diesem Ausführungsbeispiel es nicht explizit vorgesehen, dass der Ventilationsgrad "mild hyperventilated" durch eine zuvorige Eingabe des Klinikers ausgewählt werden kann, wie durch die Einträge "n/a" indiziert. Alternativ können jedoch anstelle der Einträge "n/a" Werte vorgesehen werden, so dass dann bei Auswahl des Ventilationsgrades "mild hyperventilated" entsprechende Grenzwerte der Kohlendioxidkonzentration etCO2U1, etCO2O1 ausgewählt werden können.

Die Recheneinheit R der Figur 3 ermittelt vorzugsweise auf Basis des erfassten Volumenstroms ein durch den Patienten eingeatmetes Tidalvolumen. Hierzu bestimmt die Recheneinheit vorzugsweise anhand des erfassten Volumenstroms und einer vorgegebenen Dauer T_IP der inspiratorischen Phase das Tidalvolumen als Integralwert des Volumenstroms über diese Dauer T_IP. Alternativ kann eine Zeitdauer einer inspiratorischen Phase derart ermittelt werden, dass bei Überschreiten eines Volumenstromschwellenwertes auf einen Beginn der inspiratorischen Phase geschlossen wird und bei Unterschreiten des Volumenstromschwellenwertes auf ein Ende der inspiratorischen Phase.

Die Recheneinheit bestimmt vorzugsweise auf Basis der erfassten Kohlendioxidkonzentration eine endexspiratorische Kohlendioxidkonzentration. Vorzugsweise wird durch Vergleich des Volumenstroms, wie in Figur 1 gezeigt, und eines unteren bzw. negativen USW Schwellenwertes dann auf ein Ende einer exspiratorischen Phase geschlossen, wenn der erfasste Volumenstrom den unteren Schwellenwert USW von unten her durchschreitet.

Die durch die Recheneinheit R der Figur 3 bestimmten Werte für das Tidalvolumen sowie die endexspiratorische Kohlendioxidkonzentration werden vorzugsweise alle 4 Sekunden als Messwerte bereitgestellt.

Gemäß des Schrittes S4 der Figur 4 wird zunächst 15 Sekunden lang gewartet und dann im Rahmen des Schrittes S8 vorzugsweise das in Betracht gezogene Tidalvolumen VT mittels einer Medianfilterung jener Messwerte des Tidalvolumens ermittelt, welche in den letzten 60 Sekunden vorlagen. Ebenso wird vorzugsweise die in Betracht gezogene endexspiratorische Kohlendioxidkonzentration etCO2 mittels einer Vorverarbeitung, vorzugsweise einer Medianfilterung, anhand jener Messwerte der endexspiratorischen Kohlendioxidkonzentration ermittelt, welche die letzten 60 Sekunden repräsentieren.

Es wird nun zunächst erläutert, wie vorzugsweise eine Anpassung der Beatmungsfrequenz RRmin sowie eine vorzugsweise Anpassung des Solldruckwertes ΔP in Abhängigkeit des Tidalvolumens VT und in Abhängigkeit der endexspiratorischen Kohlendioxidkonzentration etCO2 durchgeführt wird.

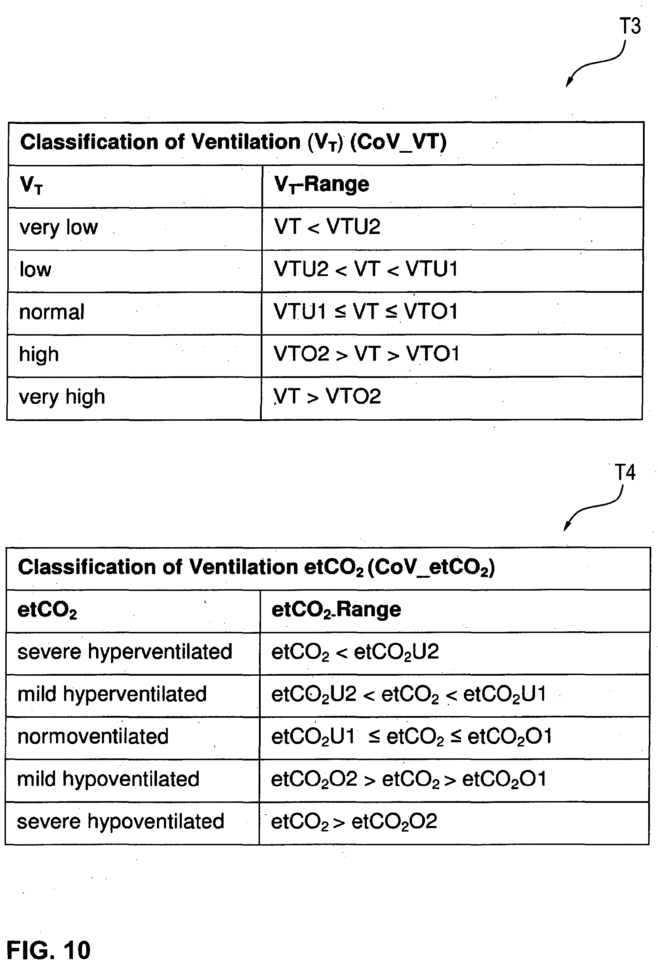

Nach Bestimmung des Tidalvolumens VT sowie der endexspiratorischen Kohlendioxidkonzentration etCO2 im Rahmen des Schrittes S8 wird vorzugsweise zunächst im Rahmen des Schrittes S9 ein Ventilationsgrad unter Bezug auf das Tidalvolumen bestimmt sowie ferner ein Ventilationsgrad in Bezug auf die endexspiratorische Kohlendioxidkonzentration. Hierzu ist die Figur 10 heranzuziehen, welche in der Tabelle T3 verschiedene Ventilationsgrade unter Vergleich des Tidalvolumens VT zu den zuvor ermittelten Grenzwerten vorgibt. Hierbei werden nicht nur der obere und der untere Grenzwert VTO1, VTU1, wie zuvor in Tabelle T2 gezeigt, herangezogen, sondern vorzugsweise auch weitere, zweite Grenzwerte VTO2, VTU2. Diese zweiten Grenzwerte VTO2, VTU2 sind ebenso in Figur 7 eingetragen. Es werden also jeweilige zweite Grenzwerte VTO2, VTU2 verwendet, bei welchen eine Abweichung um 10% bzw. 20% von den ersten Grenzwerten VTO1, VTU1 in Betracht gezogen wird.

Entsprechendes gilt für den Grad einer Ventilation in Bezug auf die endexspiratorische Kohlendioxidkonzentration in Vergleich zu den Konzentrationswerten etCO2U1, etCO2O1 sowie weiteren, zweiten Konzentrationswerten etCO2U2, etCO2O2, welche um 5% bzw. 10% von den ersten Konzentrationswerten etCO2U1, etCO2O1 abweichen und ebenfalls in Figur 7 eingetragen sind. Anhand der Tabelle T4 der Figur 10 kann also ein Ventilationsgrad in Bezug auf die endexspiratorische Kohlendioxidkonzentration bestimmt werden.

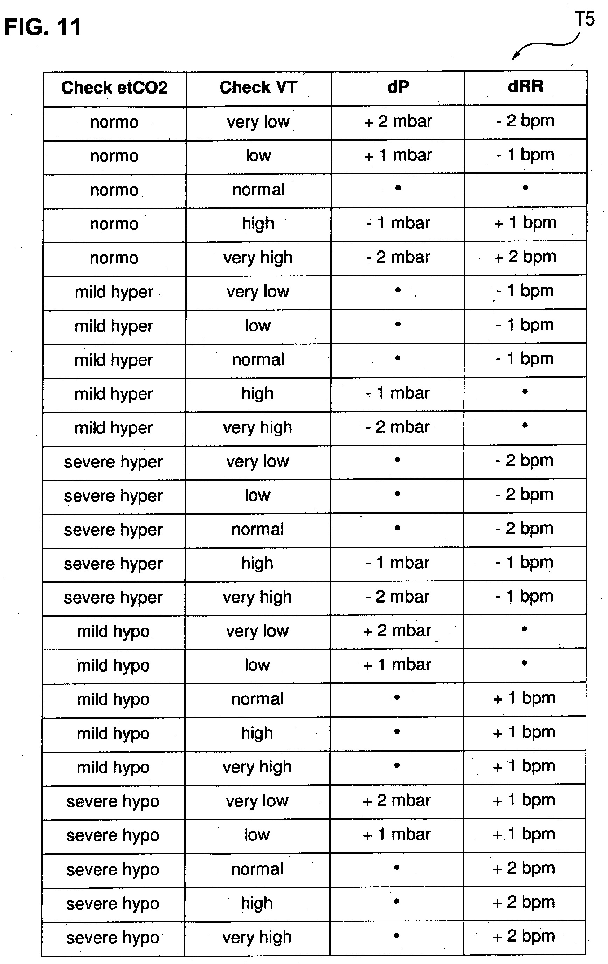

In dem in der Figur 4 gezeigten Schritt S10 erfolgt vorzugsweise eine Anpassung des Solldruckwertes ΔP sowie der minimalen Beatmungsfrequenz. Dieses erfolgt unter Zuhilfenahme einer Tabelle T5 aus der Figur 11.

Unter Hinzunahme der anhand von Tabelle T3 und Tabelle T4 der Figur 10 ermittelten Ventilationsgrade bezogen auf das Tidalvolumen VT bzw. die endexspiratorische Kohlendioxidkonzentration etCO2 kann nun anhand der Tabelle T5 der Figur 11 eine Änderung dP des Solldruckdruckwertes ΔP sowie eine Änderung dRR des Druckwertes RRmin ermittelt werden. Anhand der ermittelten Änderungen dP sowie dRR werden dann der Solldruckwert ΔP sowie die Beatmungsfrequenz RRmin angepasst. Dies erfolgt gemäß

$$\Delta P := \Delta P + dP \quad ,$$

$$RRmin := RRmin + dRR$$

Die Beatmungsfrequenz RRmin wird vorzugsweise in der zuvor beschriebenen Weise verwendet um zu detektieren, ob die Recheneinheit ein Warnsignal WS ausgeben soll.

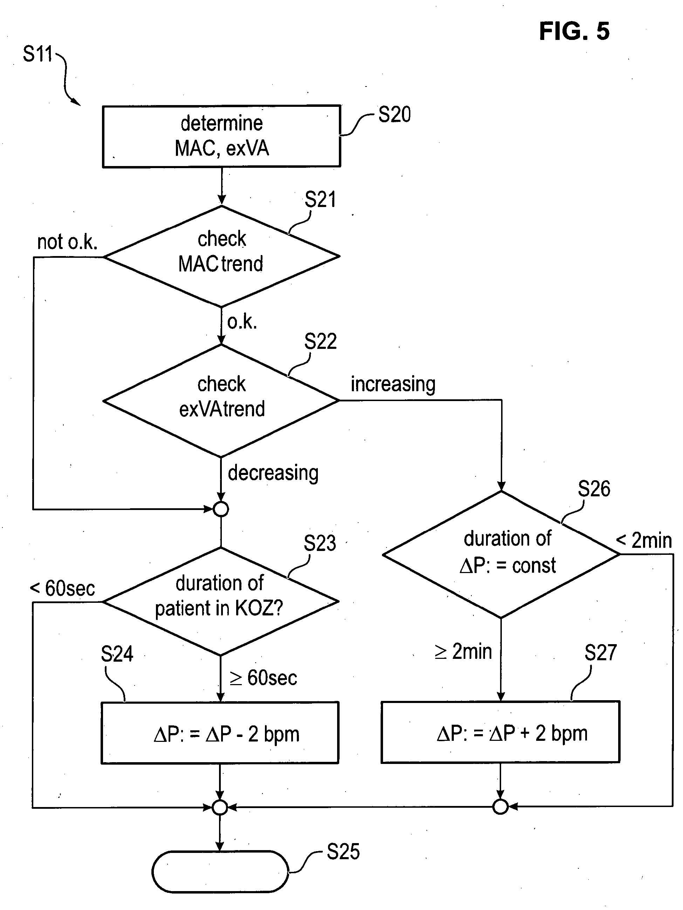

In dem Schritt S11 wird erfindungsgemäß eine Anpassung des Solldruckwertes ΔP in Abhängigkeit einer erfassten Anästhesiegaskonzentration durchgeführt. Hierzu zeigt die Figur 5 Teilschritte des Schrittes S11 aus der Figur 4.

Gemäß der Figur 5 wird in dem Teilschritt S20 zunächst eine mittlere alveoläre Anästhesiegaskonzentration MAC bestimmt. Eine solche mittlere alveoläre Anästhesiegaskonzentration wird auch "Minimum Alveolar Concentration" genannt. Diese mittlere alveoläre Anästhesiegaskonzentration MAC ist vorzugsweise eine normierte Größe xMAC, bzw. ein MAC Vielfaches, wie in der Schrift

- Primus Infinity Empowered, Dräger Medical GmbH, Ausgabe/Edition: 3/2010/09

auf den Seiten 132 - 134 genauer erläutert.

Eine solche mittlere alveoläre Anästhesiegaskonzentration MAC wird über ein gemitteltes Zeitfenster zurückliegender Messwerte, welche durch das Anästhesiegaskonzentrationssignal AGS indiziert werden, siehe Figur 3, ermittelt.

Ferner wird eine endexspiratorische Anästhesiegaskonzentration exVA ermittelt. Hierzu bestimmt die Recheneinheit R auf Basis des Anästhesiegaskonzentrationssignals AGS der Figur 3 vorzugsweise während einer endexspiratorischen Phase die Konzentration des Anästhesiegases. Hierzu kann die Recheneinheit R vorzugsweise ein Vorliegen einer exspiratorischen Phase EXP aus Figur 1 unter zu Hilfenahme des unteren Schwellenwertes USW in der zuvor genannten bzw. beschriebenen Weise detektieren und auf Messwerte der Anästhesiegaskonzentration abstellen, welche in der Endphase der exspiratorischen Phase EXP gemessen werden.

In dem Teilschritt S21 der Figur 5 wird dann überprüft, ob ein zeitlicher Verlauf der bestimmten mittleren alveolären Anästhesiegaskonzentration in ihrem zeitlichen Verlauf vorgegebene Bedingungen erfüllt. Werden diese Bedingungen nicht erfüllt, so wird zu einem Schritt S23 hin verzweigt. Werden diese Bedingungen erfüllt, so wird zu einem Schritt S23 hin verzweigt, in welchem die endexspiratorische Kohlendioxidkonzentration überprüft wird.

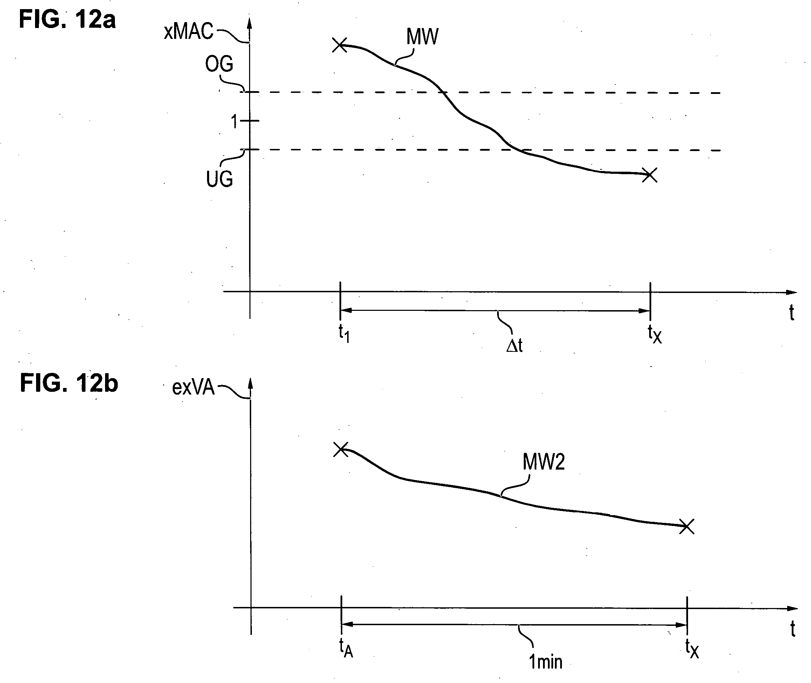

Für den Schritt S21 der Überprüfung illustriert Figur 12a einen möglichen zeitlichen Verlauf der mittleren alveolären Anästhesiegaskonzentration MAC. Messwerte MW der mittleren alveolären Anästhesiegaskonzentration MAC werden für einen aktuellen Zeitpunkt tX sowie für einen um ein vorgegebenes Zeitintervall $\Delta$t zurückliegenden Zeitpunkt t1 betrachtet. Bei der mittleren alveolären Anästhesiegaskonzentration MAC handelt es sich vorzugsweise um die zuvor erwähnte Anästhesiegaskonzentration xMAC.

Liegt für den zurückliegenden Zeitpunkt t1 die mittlere alveoläre Anästhesiegaskonzentration MAC oberhalb eines oberen Grenzwertes OG, welcher vorzugsweise für den xMAC der Wert "1,1" ist, und liegt die mittlere alveoläre Anästhesiegaskonzentration MAC unterhalb eines unteren Grenzwertes UG, welcher für den xMAC vorzugsweise der Wert "0,9" ist, so wird ein adäquates Abnehmen der mittleren alveolären Anästhesiegaskonzentration xMAC über der Zeit angenommen. Daher sind die geforderten Bedingungen dann erfüllt. Der zeitliche Verlauf der mittleren alveolären Anästhesiegaskonzentration besitzt eine zeitliche Abnahme, wie sie in einer Endphase eines Anästhesiebeatmung zu erwarten bzw. zu erzielen ist. In einer solchen Phase werden durch die Narkosegaseinheit NG der Figur 3 keine weiteren Anästhesiegase in das Atemgas eingebracht.

Zurückkommend zu der Figur 5 wird dann in dem Teilschritt S22 überprüft, ob ein zeitlicher Verlauf der endexspiratorischen Anästhesiegaskonzentration exVA ansteigend oder fallend ist. Der Teilschritt S22 der Figur 5 wird nun unter Betrachtung der Figur 12b näher erläutert.

Die Figur 12b zeigt einen möglichen zeitlichen Verlauf von Messwerten MW2 der endexspiratorischen Anästhesiegaskonzentration exVA über der Zeit t. Für einen aktuellen Zeitpunkt tX sowie für einen um einen vorgegebene Zeitraum von vorzugsweise um 1 Minute zurückliegenden Zeitpunkt tA werden die jeweiligen Messwerte in Betracht gezogen. Indiziert ein Vergleich dieser Messwerte der Zeitpunkte tX, tA eine Abnahme der endexspiratorischen Anästhesiegaskonzentration, so wird dann darauf geschlossen, dass der Patient aufgrund seines Stoffwechsels das noch in dem Atemgas vorhandene Anästhetikum abbaut bzw. reduziert. Daher wird in dem Teilschritt S22 der Figur 5 hin zu dem Teilschritt S23 der Figur 5 verzweigt. Wird jedoch anders als in Figur 12b dargestellt keine Abnahme der endexspiratorischen Anästhesiegaskonzentration festgestellt, sondern wird eine Zunahme der endexspiratorischen Anästhesiegaskonzentration detektiert, so wird von dem Teilschritt S22 hin zu einem Teilschritt S26 verzweigt.

In dem Teilschritt S23 wird detektiert, für welchen Zeitraum die Parameter des Tidalvolumens sowie der endexspiratorischen Kohlendioxidgaskonzentration innerhalb der sogenannten Komfortzone aus Figur 7 vorlagen. Ist diese Zeitdauer größer als ein vorgegebener Zeitraum von 60 Sekunden, so wird vorzugsweise dann zu dem Teilschritt S24 verzweigt. Ist diese Zeitdauer kleiner als 60 Sekunden, so wird vorzugsweise hin zu dem Teilschritt S25 verzweigt, bei welchem das Teilverfahren der Figur 5 endet. In dem Teilschritt S24 wird eine Anpassung des Solldruckwertes $\Delta$P vorgenommen, vorzugsweise gemäß

$$\Delta P := \Delta P - 2 \text{ bpm} \qquad .$$

Wurde von dem Teilschritt S22 hin zu dem Teilschritt S26 verzweigt, so wird in dem Teilschritt S26 überprüft, wie lang der Zeitraum ist, für welchen der Solldruckwert $\Delta$P konstant geblieben ist. Ist der Solldruckwert $\Delta$P für einen vorgegebenen Zeitraum von vorzugsweise 2 Minuten konstant geblieben, so wird hin zu dem Teilverfahrensschritt S27 hin verzweigt, in welchem eine Anpassung des Solldruckwertes $\Delta$P vorgenommen wird, vorzugsweise gemäß

$$\Delta P := \Delta P + 2 \text{ bpm} \qquad .$$

Ist der Solldruckwert ∆P für den vorgegebenen Zeitraum von vorzugsweise 2 Minuten nicht konstant geblieben, so wird direkt hin zu dem Teilverfahrensschritt S25 hin verzweigt

Zusammenfassend ist also unter Bezug auf den Teilschritt S11 bzw. die Figur 5 zu sagen, dass auf Basis der Anästhesiegaskonzentration eine mittlere alveoläre Anästhesiegaskonzentration sowie eine endexspiratorische Anästhesiegaskonzentration ermittelt wird, wobei eine Anpassung des Solldruckwertes ∆P in Abhängigkeit der ermittelten mittleren alveolären Anästhesiegaskonzentration sowie in Abhängigkeit der ermittelten endexspiratorischen Anästhesiegaskonzentration vorgenommen wird.

Ferner wird die Anpassung des Solldruckwertes ∆P in Abhängigkeit davon vorgenommen, ob die endexspiratorische Kohlendioxidkonzentration etCO2 und das Tidalvolumen VT für einen zurückliegenden Zeitraum, z.B. 60 Sekunden, vorgegebener Länge Grenzwertbedingungen erfüllt haben. Dieses sind die Grenzwertbedingungen, welche durch die ersten Volumengrenzwerte und die ersten Konzentrationsgrenzwerte gegeben sind. Diese Grenzwerte definieren die Komfortzone KOZ aus Figur 7.

Ferner wird die Anpassung des Solldruckwertes ∆P in Abhängigkeit davon vorgenommen, ob bei Durchführung des vorgeschlagenen Verfahrens der Solldruckwert ∆P für einen zweiten zurückliegenden Zeitraum, z.B. 2 Minuten, konstant geblieben ist.

Nach Erreichen des Teilschrittes S25 der Figur 5 ist der Verfahrensschritt S11 aus der Figur 4 zu Ende ausgeführt.

In dem Verfahrensschritt S12 der Figur 4 wird vorzugsweise detektiert, ob ein gewünschter Betriebszustand hinsichtlich der automatisierten Beatmung vorliegt. Ist dies der Fall, so kann dann zu einem Verfahrensschritt S13 fortgeführt, in welchem ein Ausgabesignal ("status OK") an einen Kliniker ausgegeben wird, welches ein Vorliegen des gewünschten Betriebszustandes indiziert. Dieses Ausgabesignal ist das Ausgabesignal AUS der Figur 3, welches durch die Recheneinheit R ausgegeben wird. Vorzugsweise erfolgt die Ausgabe dieses Ausgabesignals AUS durch die Recheneinheit R mittels einer Datenschnittstelle DAS.

Wurde nicht der gewünschte Betriebszustand detektiert, so wird von dem Verfahrensschritt S12 hin zu einem Verfahrensschritt S14 verzweigt, in welchem ein möglicherweise zuvor ausgegebenes Ausgabesignal, jenes aus dem Verfahrensschritt S13, zurückgenommen wird. Die Verfahrensschritte S13 und S14 führen das Verfahren fort, in dem dann zurückgekehrt wird zu dem Verfahrensschritt S4.

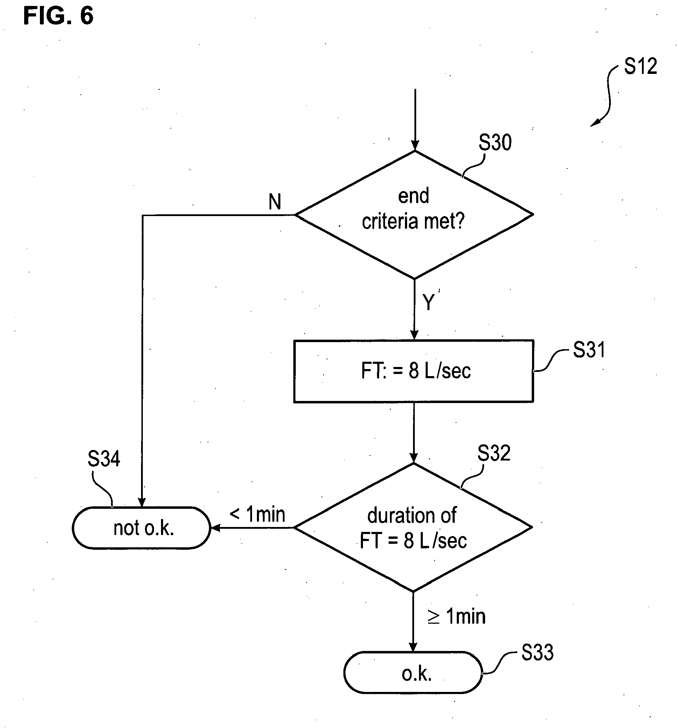

Es wird nun im Detail erläutert, in welcher Art und Weise innerhalb des Verfahrensschrittes S12 der gewünschte Betriebszustand detektiert wird. Hierzu werden Teilverfahrensschritte des Schrittes S12 anhand der Figur 6 näher erläutert.

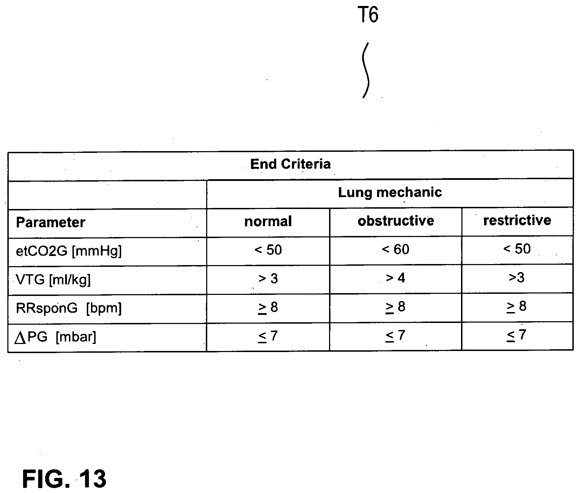

In einem Teilverfahrensschritt S30 wird überprüft, ob vorgegebene Kriterien bzw. Zielwerte erfüllt werden. Hierzu zeigt die Figur 13 eine Tabelle T6, anhand derer ein weiterer vorgegebener Konzentrationsgrenzwert für eine Kohlendioxidkonzentration etCO2G, ein weiterer vorgegebener Volumengrenzwert VTG sowie ein vorgegebener Solldruckwert ∆PG bestimmt werden können. Diese Grenzwerte bzw. Zielwerte können vorzugsweise von einer zuvor gegebenen Lungeneigenschaft ("lung mechanic") abhängen. Vorzugsweise wird auch ein Grenzwert für eine Beatmungsfrequenz RRsponG vorgegeben.

Auf Basis des durch die Recheneinheit R der Figur 3 bestimmten Tidalvolumens VT, der bestimmten endexspiratorischen Kohlendioxidkonzentration etCO2, des vorgegebenen Volumengrenzwertes VTG, des vorgegebenen Konzentrationsgrenzwertes etCO2G, des aktuellen Solldruckwertes ∆P sowie des vorgegebenen Solldruckwertes ∆PG kann nun überprüft werden, ob die zuvor genannten Zielwerte erfüllt werden. Hierzu wird vorzugsweise ferner auch eine durch die Recheneinheit R bestimmte Spontanatemaktivitätsfrequenz mit dem Grenzwert RRsponG verglichen.

Es wird in dem Teilverfahrensschritt S30 bei Erfüllen der Endkriterien bzw. der Zielwerte hin zu einem Teilverfahrensschritt S31 verzweigt wird. War die Überprüfung in dem Teilverfahrensschritt S30 negativ, so wird hin zu einem Teilverfahrensschritt S34 verzweigt, in welchem festgestellt wird, dass der gewünschte Beatmungszustand des Patienten noch nicht erreicht ist.

Wurde der gewünschte Beatmungszustand des Patienten erreicht und somit von dem Teilverfahrensschritt S30 hin zu dem Teilverfahrensschritt S31 verzweigt, so wird dort dann die Triggerschwelle FT auf einen vorgegebenen Wert, von vorzugsweise 8 Liter/s, gesetzt.

In einem darauf folgenden Teilverfahrensschritt S32 wird dann überprüft, ob das Beatmungssystem bzw. die Beatmungsvorrichtung die druckunterstützende Beatmung des Patienten erfolgreich für einen vorgegebenen Zeitraum, vorzugsweise 1 Minute, mit dem vorgegebenen Schwellenwert von 8 L/s durchgeführt hat, sodass kein Warnsignal ausgegeben wurde, welches indiziert, dass eine Mindestbeatmungsfrequenz RRmin durch die Beatmungsfrequenz des Patienten aufgrund seiner Spontanatemaktivität unterschritten wurde.

War dies nicht erfolgreich, so wird von dem Teilverfahrensschritt S32 hin zu dem Teilverfahrensschritt S34 verzweigt, in welchem detektiert wird, dass der gewünschte Beatmungszustand des Patienten nicht vorliegt.

Wurde festgestellt, dass der Patient erfolgreich unter Berücksichtigung der Triggerschwelle FT in druckunterstützender Weise für den vorgegebenen Zeitraum, vorzugsweise 1 Minute, beatmet wurde, so wird von dem Teilverfahrensschritt S32 hin zu dem Teilverfahrensschritt S33 verzweigt, wobei in dem Teilverfahrensschritt S33 das Vorliegen des gewünschten Betriebszustandes detektiert wird.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks/Schrittes oder Details oder Merkmals einer entsprechenden Vorrichtung dar, bzw. dass die Vorrichtung oder die entsprechende Recheneinheit zur Durchführung des Verfahrensschrittes ausgebildet ist.

Die in Figur 3 gezeigte Recheneinheit R ist als wenigstens eine Recheneinheit anzusehen. Eine Umsetzung der wenigstens einen Recheneinheit R kann auch durch eine Kombination mehrerer Recheneinheiten verwirklicht werden, vorzugsweise durch Verwendung von Software in Verbindung mit Hardware. Je nach bestimmten Implementierungsanforderungen können Ausführungsbeispiele der Erfindung in Hardware und/oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardware-Komponente derart zusammenwirken können oder zusammenwirken, dass das jeweilige Verfahren durchgeführt wird.

Eine programmierbare Hardware-Komponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikroprozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbare Hardware-Komponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardware-Komponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

### Bezugszeichenliste

| | |
|---|---|
| Anzeigeeinheit | AE |
| Atemgasfördereinheit | AGF |

(fortgesetzt)

| | |
|---|---|
| Ausgabesignal | AUS |
| Anästhesiegasfortleitung | ANF |
| Anästhesiegaskonzentrationssignal | AGS |
| Ansteuersignal | ANS |
| Atemgassensor | AS |
| Beatmungsschlauch | BS |
| Beatmungsvorrichtung | BV |
| Kohlendioxidabsorber | CA |
| Datenschnittstelle | DAS |
| Änderung des Druckwertes ∆P | dP |
| Änderungsgrad der Beatmungsfrequenz RR | dRR |
| Drucksensor | DS |
| Drucksensorsignal | DSS |
| Eingabeeinheit | EE |
| Exspiratorischer Port | EP |
| endexspiratorischen Kohlendioxidkonzentration | etCO2 |
| Grenzwert für Kohlendioxidkonzentration | etCO2G |
| oberer Konzentrationsgrenzwert | etCO2O1, etCO2O2 |
| unterer Konzentrationsgrenzwert | etCO2U1, etCO2U2 |
| Exspiratorische Phase | EXP |
| exspiratorische Anästhesiegaskonzentration | exVA |
| Beatmungsfrequenz | F, RR |
| Mindestatemfrequenz | Fmin, RRmin |
| Triggerschwelle | FT |
| Inspiratorische Phase | INP |
| Inspiratorischer Port | IP |
| Kolbeneinheit | KE |
| Kolben | KO |
| Komfortzone | KOZ |
| Kohlendioxidkonzentrationssignal | KSS |
| Messleitung | LT |
| Messport | LTP |
| Motor | M |
| Modus | M1, M2, M3 |
| mittlere alveoläre Anästhesiegaskonzentration | MAC, xMAC |
| Speichereinheit | MEM |
| Messwerte | MW, MW2 |
| Narkosegasmischeinheit | NG |
| Steuersignal | NGAS |

(fortgesetzt)

| | |
|---|---|
| oberer Grenzwert | OG |
| Druck | P |
| Mindestdruck | PEEP |
| Maximaldruckwert | Pinsp |
| Maximaldruck | Pmax |
| Patient | PT |
| Ventil | PV |
| Recheneinheit | R |
| Grenzwert für Spontanatemaktivitätsfrequenz | RRsponG |
| Rückschlagventile | RV |
| Schritt | S1, ... , S14 |
| Teilverfahrensschritt | S20, ... , S27; S30, ... , S34 |
| Spalte | SP1, SP2 |
| Zeit | t |
| Zeitlicher Abstand | T, $\Delta t$ |
| Tabelle | T1, ... , T6 |
| Zeitdauer | T_IP, Tmax |
| Zeitpunkt | tX, t1, tA, ZP |
| unterer Grenzwert | UG |
| unterer Schwellenwert | USW |
| Volumenstrom | V |
| Volumenstromsensor | VS |
| Volumenstromsensorsignal | VSS |
| Tidalvolumen | VT |
| vorgegebener Volumengrenzwert | VTG |
| oberer Volumengrenzwert | VTO1, VTO2 |
| unterer Volumengrenzwert | VTU1, VTU2 |
| Warnsignal | WS |
| Warnsignalausgabeeinheit | WSE |
| Y-Stück | YS |
| Differenzdruck | $\Delta P$ |
| vorgegebener Solldruckwert | $\Delta PG$ |

**Patentansprüche**

1. Anästhesiebeatmungsvorrichtung (BV) zur automatisierten Beatmung eines Patienten (PT), aufweisend

- einen exspiratorischen Port (EP) und einen inspiratorischen Port (IP) zum Anschluss eines dem Patienten (PT) zugewandten Beatmungsschlauches (BS) für ein Atemgas,
- eine Atemgasfördereinheit (AGF),

- wenigstens einen Atemgassensor (AS) zum Erfassen einer Anästhesiegaskonzentration,
- wenigstens einen Drucksensor (DS) zum Erfassen eines Drucks des Atemgases,
- sowie wenigstens eine Recheneinheit (R),
wobei die Recheneinheit (R) dazu ausgebildet ist,
- die Atemgasfördereinheit (AGF) in Abhängigkeit des erfassten Drucks (P) und eines vorgegebenen Solldruckwertes (ΔP) anzusteuern,

**dadurch gekennzeichnet, dass** die Recheneinheit (R) ferner dazu ausgebildet ist, eine Anpassung des Solldruckwertes (ΔP) in Abhängigkeit der erfassten Anästhesiegaskonzentration vorzunehmen.

2. Anästhesiebeatmungsvorrichtung (BV) nach Anspruch 1,
**dadurch gekennzeichnet, dass** die Recheneinheit (R) dazu ausgebildet ist, auf Basis der erfassten Anästhesiegaskonzentration eine mittlere alveoläre Anästhesiegaskonzentration sowie eine endexspiratorische Anästhesiegaskonzentration zu ermitteln,
und dass die Recheneinheit (R) ferner dazu ausgebildet ist, eine Anpassung des Solldruckwertes (ΔP) in Abhängigkeit der ermittelten mittleren alveolären Anästhesiegaskonzentration sowie in Abhängigkeit der ermittelten endexspiratorischen Anästhesiegaskonzentration vorzunehmen.

3. Anästhesiebeatmungsvorrichtung (BV) nach Anspruch 1,
**dadurch gekennzeichnet, dass** die Vorrichtung ferner

- wenigstens einen Volumenstromsensor (VS) zum Erfassen eines Volumenstroms (V) des Atemgases
- sowie ferner wenigstens einen Atemgassensor (AS) zum Erfassen einer Kohlendioxidkonzentration des Atemgases

aufweist,
**dadurch gekennzeichnet, dass** die Recheneinheit (R) ferner dazu ausgebildet ist, eine Anpassung des Solldruckwertes (ΔP) in Abhängigkeit des erfassten Volumenstromes (V) und in Abhängigkeit der erfassten Kohlendioxidkonzentration vorzunehmen.

4. Anästhesiebeatmungsvorrichtung (BV) nach Anspruch 3,
**dadurch gekennzeichnet, dass** die Recheneinheit (R) dazu ausgebildet ist

- auf Basis des erfassten Volumenstroms (V) ein dem Patienten (PT) zugeführtes Tidalvolumen (VT) zu bestimmen
- und ferner auf Basis der erfassten Kohlendioxidkonzentration eine endexspiratorische Kohlendioxidkonzentration (etCO2) zu bestimmen,
und dass die Recheneinheit (R) ferner dazu ausgebildet ist, die Anpassung des Solldruckwertes (ΔP) sowie einer minimalen Beatmungsfrequenz (RRmin) ferner in Abhängigkeit
- des bestimmten Tidalvolumens (VT),
- eines oberen Volumengrenzwertes (VTO1)
- und eines unteren Volumengrenzwertes (VTU1)
- der bestimmten endexspiratorischen Kohlendioxidkonzentration (etCO2),
- eines oberen Konzentrationsgrenzwertes (etCO2O1)
- und eines unteren Konzentrationsgrenzwertes (etCO2U1) vorzunehmen.

5. Anästhesiebeatmungsvorrichtung nach Anspruch 4,
**dadurch gekennzeichnet, dass** die Recheneinheit (R) ferner dazu ausgebildet ist,
in Abhängigkeit

- des bestimmten Tidalvolumens (VT),
- der bestimmten endexspiratorischen Kohlendioxidkonzentration (etCO2),
- eines weiteren vorgegebenen Volumengrenzwertes (VTG),
- eines weiteren vorgegebenen Konzentrationsgrenzwertes (etCO2G),
- des aktuellen Solldruckwertes (ΔP) sowie
- eines vorgegebenen Solldruckgrenzwertes (ΔPG)

einen gewünschten Betriebszustand hinsichtlich der automatisierten Beatmung zu detektieren und bei Detektion ein Ausgabesignal an einen Kliniker auszugeben, welches ein Vorliegen des gewünschten Betriebszustandes indiziert.

6. Anästhesiebeatmungsvorrichtung (BV) nach Anspruch 1,
**dadurch gekennzeichnet, dass** die Recheneinheit (R) dazu ausgebildet ist, die Atemgasfördereinheit (AGF) so anzusteuern, dass die automatisierte Beatmung als eine druckunterstützende Beatmung durchgeführt wird.

7. Anästhesiebeatmungsvorrichtung (BV) nach Anspruch 6,
**dadurch gekennzeichnet, dass** die Vorrichtung ferner wenigstens einen Volumenstromsensor (VS) zum Erfassen eines Volumenstroms (V) des Atemgases aufweist,
und dass ferner die Recheneinheit ferner dazu ausgebildet ist,

- auf Basis des erfassten Volumenstroms (V) einen Spontanatemversuch des Patienten (PT) zu detektieren,
- und ferner die druckunterstützende Beatmung unter Verwendung des Solldruckwertes (ΔP) bei Detektion eines Spontanatemversuchs durchzuführen.

8. Anästhesiebeatmungsvorrichtung (BV) nach Anspruch 7,
**dadurch gekennzeichnet, dass** die Recheneinheit (R) ferner dazu ausgebildet ist, in Abhängigkeit detektierter Spontanatemversuche und in Abhängigkeit einer vorgebbaren minimalen Beatmungsfrequenz (RRmin) eine Ausgabe eines Warnsignals (WS) zu steuern.

9. Recheneinheit (R) für eine Anästhesiebeatmungsvorrichtung (BV) zur automatisierten Beatmung eines Patienten (PT), ausgebildet zum

- Erfassen eines Anästhesiegaskonzentrationssignals (AGS), welches eine Anästhesiegaskonzentration eines Atemgases indiziert,
- Erfassen eines Drucksignals (DSS), welches einen Druck (P) des Atemgases indiziert,
- Bereitstellen eines Ansteuersignals (ANS) für eine Atemgasfördereinheit (AGF), wobei die Recheneinheit (R) das Ansteuersignal (ANS) in Abhängigkeit des erfassten Drucksignals (DSS) und eines vorgegebenen Solldruckwertes (ΔP) bestimmt, **dadurch gekennzeichnet, dass** die Recheneinheit (R) ausgebildet ist, eine Anpassung des Solldruckwertes (ΔP) in Abhängigkeit des erfassten Anästhesiegaskonzentrationssignals (AGS) vorzunehmen.

## Claims

1. Anaesthesia ventilator (BV) for the automated ventilation of a patient (PT),
having

- an expiratory port (EP) and an inspiratory port (IP) for the attachment of a ventilation tube (BS) directed towards the patient (PT) and supplying a breathing gas,
- a breathing gas delivery unit (AGF),
- at least one breathing gas sensor (AS) for detecting an anaesthetic gas concentration,
- at least one pressure sensor (DS) for detecting a pressure of the breathing gas,
- and at least one computer (R),
wherein the computer (R) is configured
- to actuate the breathing gas delivery unit (AGF) as a function of the detected pressure (P) and of a preset desired pressure value (ΔP),

**characterized in that** the computer (R) is moreover configured to effect an adaptation of the desired pressure value (ΔP) as a function of the detected anaesthetic gas concentration.

2. Anaesthesia ventilator (BV) according to Claim 1,
**characterized in that** the computer (R) is configured to determine, on the basis of the detected anaesthetic gas concentration, a mean alveolar anaesthetic gas concentration and also an end-expiratory anaesthetic gas concentration,
and **in that** the computer (R) is moreover configured to effect an adaptation of the desired pressure value (ΔP) as a function of the determined mean alveolar anaesthetic gas concentration and also as a function of the determined end-expiratory anaesthetic gas concentration.

3. Anaesthesia ventilator (BV) according to Claim 1,
   **characterized in that** the ventilator moreover has

   - at least one volume flow sensor (VS) for detecting a volume flow (V) of the breathing gas
   - and moreover at least one breathing gas sensor (AS) for detecting a carbon dioxide concentration of the breathing gas,

   **characterized in that** the computer (R) is moreover configured to effect an adaptation of the desired pressure value (ΔP) as a function of the detected volume flow (V) and as a function of the detected carbon dioxide concentration.

4. Anaesthesia ventilator (BV) according to Claim 3,
   **characterized in that** the computer (R) is configured

   - to determine, on the basis of the detected volume flow (V), a tidal volume (VT) delivered to the patient (PT),
   - and moreover to determine, on the basis of the detected carbon dioxide concentration, an end-expiratory carbon dioxide concentration (etCO2),
   and **in that** the computer (R) is moreover configured to effect the adaptation of the desired pressure value (ΔP) and also of a minimum ventilation rate (RRmin) moreover as a function of
   - the determined tidal volume (VT),
   - an upper volume limit value (VTO1)
   - and a lower volume limit value (VTU1)
   - the determined end-expiratory carbon dioxide concentration (etCO2),
   - an upper concentration limit value (etCO2O1)
   - and a lower concentration limit value (etCO2U1).

5. Anaesthesia ventilator according to Claim 4,
   **characterized in that** the computer (R) is moreover configured to detect a desired operating state as regards the automated ventilation as a function of

   - the determined tidal volume (VT),
   - the determined end-expiratory carbon dioxide concentration (etCO2),
   - a further preset volume limit value (VTG)
   - a further preset concentration limit value (etCO2G),
   - the current desired pressure value (ΔP) and
   - a preset desired pressure limit value (ΔPG)

   and, in the event of detection, to output to a clinician an output signal which indicates the presence of the desired operating state.

6. Anaesthesia ventilator (BV) according to Claim 1,
   **characterized in that** the computer (R) is configured to actuate the breathing gas delivery unit (AGF) such that the automated ventilation is carried out as pressure support ventilation.

7. Anaesthesia ventilator (BV) according to Claim 6,
   **characterized in that** the ventilator moreover has at least one volume flow sensor (VS) for detecting a volume flow (V) of the breathing gas,
   and moreover **in that** the computer is moreover configured

   - to detect a spontaneous ventilation attempt by the patient (PT) on the basis of the detected volume flow rate (V),
   - and moreover to carry out the pressure support ventilation using the desired pressure value (ΔP) if an attempt at spontaneous ventilation is detected.

8. Anaesthesia ventilator (BV) according to Claim 7,
   **characterized in that** the computer (R) is moreover configured to control an output of a warning signal (WS) as a function of detected spontaneous ventilation attempts and as a function of a presettable minimum ventilation rate (RRmin).

**9.** Computer (R) for an anaesthesia ventilator (BV) for the automated ventilation of a patient (PT), said computer being configured

- to detect an anaesthetic gas concentration signal (AGS), which indicates an anaesthetic gas concentration of a breathing gas,
- to detect a pressure signal (DSS), which indicates a pressure (P) of the breathing gas,
- to provide an actuation signal (ANS) for a breathing gas delivery unit (AGF), wherein the computer (R) determines the actuation signal (ANS) as a function of the detected pressure signal (DSS) and of a preset desired pressure value ($\Delta$P),

**characterized in that** the computer (R) is configured to effect an adaptation of the desired pressure value ($\Delta$P) as a function of the detected anaesthetic gas concentration signal (AGS).

**Revendications**

**1.** Dispositif d'anesthésie ventilatoire (BV), destiné à la ventilation automatisée d'un patient (PT), comportant

- un port expiratoire (EP) et un port inspiratoire (IP) destinés à connecter un flexible de ventilation (BS) dirigé vers le patient (PT) pour un gaz respiratoire,
- une unité de transport de gaz respiratoire (AGF),
- au moins un capteur de gaz respiratoire (AS) destiné à détecter une concentration de gaz anesthésiant,
- au moins un capteur de pression (DS) destiné à détecter une pression du gaz respiratoire,
- ainsi qu'au moins une unité de calcul (R),
l'unité de calcul (R) étant réalisée pour
- activer l'unité de transport de gaz respiratoire (AGF) en fonction de la pression (P) détectée et d'une valeur de pression de consigne ($\Delta$P) prédéfinie,

**caractérisé en ce que** l'unité de calcul (R) est réalisée par ailleurs pour procéder à une adaptation de la valeur de pression de consigne ($\Delta$P), en fonction de la concentration de gaz anesthésiant détectée.

**2.** Dispositif d'anesthésie ventilatoire (BV) selon la revendication 1, **caractérisé en ce que** l'unité de calcul (R) est réalisée pour déterminer, sur la base de la concentration de gaz anesthésiant détectée, une concentration alvéolaire moyenne de gaz anesthésiant ainsi qu'une concentration de gaz anesthésiant résiduelle expiratoire, et **en ce que** l'unité de calcul (R) est réalisée par ailleurs pour procéder à une adaptation de la valeur de pression de consigne ($\Delta$P) en fonction de la concentration alvéolaire moyenne de gaz anesthésiant déterminée, ainsi qu'en fonction de la concentration de gaz anesthésiant résiduelle expiratoire déterminée.

**3.** Dispositif d'anesthésie ventilatoire (BV) selon la revendication 1, **caractérisé en ce que** le dispositif comporte par ailleurs

- au moins un capteur de débit volumétrique (VS) destiné à détecter un débit volumétrique (V) du gaz respiratoire,
- ainsi que par ailleurs, au moins un capteur de gaz respiratoire (AS) destiné à détecter une concentration de dioxyde de carbone du gaz respiratoire,

**caractérisé en ce que** l'unité de calcul (R) est réalisée par ailleurs pour procéder à une adaptation de la valeur de pression de consigne ($\Delta$P) en fonction du débit volumétrique (V) détecté et en fonction de la concentration de dioxyde de carbone détectée.

**4.** Dispositif d'anesthésie ventilatoire (BV) selon la revendication 3, **caractérisé en ce que** l'unité de calcul (R) est réalisée

- pour déterminer, sur la base du débit volumétrique (V) détecté, un volume courant (VT) alimenté vers le patient (PT),
- et par ailleurs, pour déterminer, sur la base de la concentration de dioxyde de carbone détectée, une concentration de dioxyde de carbone (etCO2) résiduelle expiratoire,
et **en ce que** l'unité de calcul (R) est réalisée par ailleurs pour procéder à l'adaptation de la valeur de pression de consigne ($\Delta$P) ainsi que d'une fréquence minimale de ventilation (RRmin) en outre en fonction

- du volume courant (VT) déterminé,
- d'une valeur limite de volume supérieure (VTO1)
- et d'une valeur limite de volume inférieure (VTU1)
- de la concentration de dioxyde de carbone (etCO2) résiduelle expiratoire déterminée,
- d'une valeur limite de concentration supérieure (etCO2O1)
- et d'une valeur limite de concentration inférieure (etCO2U1).

5. Dispositif d'anesthésie ventilatoire selon la revendication 4, **caractérisé en ce que** l'unité de calcul (R) est réalisée par ailleurs pour détecter, en fonction

- du volume courant (VT) déterminé,
- de la concentration de dioxyde de carbone (etCO2) résiduelle expiratoire déterminée,
- d'une valeur limite de volume (VTG) prédéfinie supplémentaire,
- d'une valeur limite de concentration (etCO2G) prédéfinie supplémentaire,
- de la valeur de pression de consigne (ΔP) actuelle, ainsi que
- d'une valeur limite de pression de consigne (ΔPG) prédéfinie,

un état de service souhaité au niveau de la ventilation automatisée et lors de la détection, pour délivrer un signal de sortie à l'attention d'un clinicien, lequel indexe la présence de l'état de service souhaité.

6. Dispositif d'anesthésie ventilatoire (BV) selon la revendication 1, **caractérisé en ce que** l'unité de calcul (R) est réalisée pour activer l'unité de transport de gaz respiratoire (AGF), de sorte que la ventilation automatisée soit réalisée sous la forme d'une ventilation à aide inspiratoire.

7. Dispositif d'anesthésie ventilatoire (BV) selon la revendication 6, **caractérisé en ce que** le dispositif comporte par ailleurs au moins un capteur de débit volumétrique (VS) destiné à détecter un débit volumétrique (V) du gaz respiratoire,
et **en ce qu'**en outre, l'unité de calcul est réalisée par ailleurs,

- pour détecter sur la base du débit volumétrique (V) détecté une tentative respiratoire spontanée du patient (PT),
- et pour procéder par ailleurs à la ventilation à aide inspiratoire en utilisant la valeur de pression de consigne (ΔP), lors de la détection d'une tentative respiratoire spontanée.

8. Dispositif d'anesthésie ventilatoire (BV) selon la revendication 7, **caractérisé en ce que** l'unité de calcul (R) est réalisée par ailleurs, pour commander, en fonction de tentatives respiratoires spontanées détectées et en fonction d'une fréquence minimale de ventilation (RRmin) prédéfinissable, une délivrance d'un signal d'alerte (WS).

9. Unité de calcul (R), destinée à un dispositif d'anesthésie ventilatoire (BV) pour la ventilation automatisée d'un patient (PT), réalisée pour

- détecter un signal de concentration de gaz anesthésiant (AGS), lequel indexe une concentration de gaz anesthésiant d'un gaz respiratoire,
- détecter un signal de pression (DSS), lequel indexe une pression (P) du gaz respiratoire,
- mettre à disposition un signal d'activation (ANS) pour une unité de transport de gaz respiratoire (AGF), l'unité de calcul (R) déterminant le signal d'activation (ANS) en fonction du signal de pression (DSS) détecté et d'une valeur de pression de consigne (ΔP) prédéfinie, **caractérisée en ce que** l'unité de calcul (R) est réalisée pour procéder à une adaptation de la valeur de pression de consigne (ΔP) en fonction du signal de concentration de gaz anesthésiant (AGS) détecté.

**FIG. 1**

EP 3 383 467 B1

$$\frac{1}{f_{min}} \triangleq Tmax \qquad f_{min} = RRmin$$

**FIG. 2**

FIG. 3

EP 3 383 467 B1

**FIG. 4**

```
                    ┌──────────────┐
                    │    Input     │ ─── S1
                    └──────┬───────┘
                           │
              ┌────────────────────────┐
              │  RRmin: = 8/min        │
              │  ΔP: = 5 mbar          │ ─── S2
              │  PEEP: = 5 mbar        │
              │  FT: = 3 L/sec         │
              └────────────┬───────────┘
                           │
                    ┌──────────────┐
                    │   set KOZ    │ ─── S3
                    └──────┬───────┘
                           │
                           ○ ◄──────────────────────┐
                           │                         │
                    ┌──────────────┐                 │
              ⧗     │    15 sec    │ ─── S4          │
                    └──────┬───────┘                 │
                           │                         │
          N        ╱───────────────╲  ─── S5         │
      ┌────────────   VTspon?       │                │
      │            ╲───────┬───────╱                 │
     S6                    │ Y                        │
      │            ┌──────────────┐                  │
   ┌──────┐        │  determine   │ ─── S8           │
   │  X   │        │  VT, etCO₂   │                  │
   └──┬───┘        └──────┬───────┘                  │
      │                   │                          │
 ┌─────────┐       ┌──────────────┐                  │
 │ failed  │       │    CoV       │ ─── S9           │
 └─────────┘       └──────┬───────┘                  │
      │                   │                          │
     S7            ┌──────────────┐                  │
                   │ set ΔP, RRmin│ ─── S10          │
                   └──────┬───────┘                  │
                          │                          │
                   ┌──────────────┐                  │
                   │   Weaning    │ ─── S11          │
                   └──────┬───────┘                  │
         S12              │            S14           │
          ╱───────────────╲   not o.k.  ┌──────┐    │
          │  check status   ───────────►│  X   │───►○
          ╲───────┬───────╱             └──────┘
                  │ o.k.
          ┌──────────────┐ ─── S13
          │ "status o.k." │──────────────────────────┘
          └──────────────┘
```

**FIG. 5**

## FIG. 6

FIG. 7

T1

| Input | Valid Range | |
|---|---|---|
| Level of ventilation | normal ventilated, mild hyperventilated | SP1 |
| Lung mechanic | normal, restrictive, obstructive | SP2 |

**FIG. 8**

T2

| Level of ventilation | | | |
|---|---|---|---|
| normal ventilated | Lung mechanic | | |
| KOZ Parameter | normal | obstructive | restrictive |
| etCO$_2$U1 [mmHg] | 45 | 55 | 45 |
| etCO$_2$O1 [mmHg] | 55 | 65 | 55 |
| Level of ventilation | | | |
| mild hyperventilated | Lung mechanic | | |
| KOZ Parameter | normal | obstructive | restrictive |
| etCO$_2$U1 [mmHg] | n/a | n/a | n/a |
| etCO$_2$O1 [mmHg] | n/a | n/a | n/a |
| | Lung mechanic | | |
| KOZ Parameter | normal | obstructive | restrictive |
| VTU1 [ml/kg] | 4 | 4 | 2 |
| VTO1 [ml/kg] | 6 | 6 | 4 |

# FIG. 9

T3

| Classification of Ventilation ($V_T$) (CoV_VT) | |
|---|---|
| $V_T$ | $V_T$-Range |
| very low | VT < VTU2 |
| low | VTU2 < VT < VTU1 |
| normal | VTU1 ≤ VT ≤ VTO1 |
| high | VTO2 > VT > VTO1 |
| very high | VT > VTO2 |

T4

| Classification of Ventilation $etCO_2$ (CoV_$etCO_2$) | |
|---|---|
| $etCO_2$ | $etCO_2$-Range |
| severe hyperventilated | $etCO_2$ < $etCO_2$U2 |
| mild hyperventilated | $etCO_2$U2 < $etCO_2$ < $etCO_2$U1 |
| normoventilated | $etCO_2$U1 ≤ $etCO_2$ ≤ $etCO_2$O1 |
| mild hypoventilated | $etCO_2$O2 > $etCO_2$ > $etCO_2$O1 |
| severe hypoventilated | $etCO_2$ > $etCO_2$O2 |

## FIG. 10

# FIG. 11

T5

| Check etCO2 | Check VT | dP | dRR |
|---|---|---|---|
| normo | very low | + 2 mbar | - 2 bpm |
| normo | low | + 1 mbar | - 1 bpm |
| normo | normal | • | • |
| normo | high | - 1 mbar | + 1 bpm |
| normo | very high | - 2 mbar | + 2 bpm |
| mild hyper | very low | • | - 1 bpm |
| mild hyper | low | • | - 1 bpm |
| mild hyper | normal | • | - 1 bpm |
| mild hyper | high | - 1 mbar | • |
| mild hyper | very high | - 2 mbar | • |
| severe hyper | very low | • | - 2 bpm |
| severe hyper | low | • | - 2 bpm |
| severe hyper | normal | • | - 2 bpm |
| severe hyper | high | - 1 mbar | - 1 bpm |
| severe hyper | very high | - 2 mbar | - 1 bpm |
| mild hypo | very low | + 2 mbar | • |
| mild hypo | low | + 1 mbar | • |
| mild hypo | normal | • | + 1 bpm |
| mild hypo | high | • | + 1 bpm |
| mild hypo | very high | • | + 1 bpm |
| severe hypo | very low | + 2 mbar | + 1 bpm |
| severe hypo | low | + 1 mbar | + 1 bpm |
| severe hypo | normal | • | + 2 bpm |
| severe hypo | high | • | + 2 bpm |
| severe hypo | very high | • | + 2 bpm |

FIG. 12a

FIG. 12b

T6

| End Criteria | | | |
|---|---|---|---|
| | **Lung mechanic** | | |
| **Parameter** | **normal** | **obstructive** | **restrictive** |
| etCO2G [mmHg] | < 50 | < 60 | < 50 |
| VTG [ml/kg] | > 3 | > 4 | >3 |
| RRsponG  [bpm] | ≥ 8 | ≥ 8 | ≥ 8 |
| $\triangle$PG  [mbar] | ≤ 7 | ≤ 7 | ≤ 7 |

# FIG. 13

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 69630181 T2
- US 6131571 A
- EP 2572748 B1
- DE 19751597 A1
- DE 102011106406 A1

**In der Beschreibung aufgeführte Nicht-Patentliteratur**

- **KARIN DEDEN.** Beatmungsmodi in der Intensiv-medizin. Dräger Medical GmbH
- Gebrauchsanweisung. Zeus Infinity Empowered. Dräger Medical AG & Co. KG, Februar 2009
- Primus Infinity Empowered. Dräger Medical GmbH, 03. September 2010, 132-134